# EUROPEAN PATENT APPLICATION

(11) **EP 4 223 225 A1**
(43) Date of publication of application: **09.08.2023**
(21) Application number: 22155120.3
(22) Date of filing: 04.02.2022
(51) Int. Cl.: A61B 6/00, G06F 3/048

(54) **COMPUTER IMPLEMENTED METHOD FOR DISPLAYING VISUALIZABLE DATA, COMPUTER PROGRAM AND USER INTERFACE**

(71) Applicant: Koninklijke Philips N.V., 5656 AG Eindhoven (NL)
(72) Inventor: SCHRECKENBERG, Marcus, Eindhoven (NL); ZOERNACK, Sabrina, Eindhoven (NL)
(74) Representative: Philips Intellectual Property & Standards

(57) **Abstract**

A Computer implemented method for displaying visualizable data including at least one structure of interest is provided. The method comprising: - receiving a user input via a user interface (1), the user interface (1) allowing the user (100) to select
- at least one structure, and/or
- at least one view of at least one structure, and/or
- at least one function and/or
- at least one time-interval and/or
- at least one time-dependent measurement, and/or
- a reading stack and/or
- a modality
with a single command issued by the user (100) so as to generate user input data, - determining a presentation sequence of the visualizable data based on the user input data, and - displaying at least some of the visualizable data according to the presentation sequence of the visualizable data. Further, a computer program and a user interface are provided.

## Description

### FIELD OF THE INVENTION

The present invention relates to a computer implemented method for displaying visualizable data, a computer program and a user interface.

### BACKGROUND OF THE INVENTION

Visualizable data is often used to assess or to examine structures such as anatomical structures, industrial structures, etc. This means that visualizable data is collected in advance and then examined. In the medical field, visualizable data is used for an image-based diagnosis, for example. Such assessment or examination is carried out to answer to specific questions or tasks of a user. Such question or task may be a medical diagnosis, measurements, a specific status of the structure or properties of a structure. As a result, only a specific part of the available visualizable data is necessary to answer to the specific question or task posed in the most cases. Particularly in clinical applications, a large amount of visualizable data may be available, with only a small part of it being at all useful to answer the question posed.

The usual workflow for examining visualizable data is to search the image(s) the user wants to work with (e.g., to measure on), then select the wished action (e.g., a measurement) from a hierarchical menu and finally perform the desired action process.

The problem with that is that much time is lost during searching of the suitable images and then again when clicking oneself through an often very long and deeply nested menu providing a plurality of actions. For example, the best image for a measurement (where structures are visible in the best way) might be overlooked and an unfavourable one might be used for the measurement. This could lead to incorrect measurements.

In particular, a usual workflow for working with clinical images exhibits a gap between clinical and technical thinking. Naturally, a physician first has an idea of an anatomical structure in mind that shall be measured and/or diagnosed. This is translated to a technical method configured to perform the respective action (e.g., a measurement) as follows: to perform a measurement, the suitable data must be looked up within a large amount of visualizable data. Next, the desired measurement must be selected from a usually large and deeply nested, hierarchical menu of different measurements. Finally, the measurement can be performed.

There are several problems in this approach. It can take a long time to scroll through all available visualizable data to find the suitable data for answering to the currently posed question or task (e.g., searching for the visualizable data that includes the desired structure such as a left ventricle, view such as a parasternal long axis view or function such as diastolic function). This is especially true in case the visualizable data is presented to the user in several pages. By searching for the suitable visualizable data, the best fitting visualizable data might be overlooked and a suboptimal one could be chosen. This could lead to incorrect assessments or measurements. To select the desired measurement to be executed within the visualizable data is also time consuming, as mostly there is a hierarchical menu that needs several clicks to find and select the measurement the user is searching for. Furthermore, the user has to perform unnatural ways of thinking to get from his posed question (e.g., "I want to measure the left ventricle") to the actual measurement value. That is, the user must take the indirect route of "which data do I need?" over "where is this measurement situated in the menu?" to finally answer the user s question.

US 2008232661 A1 shows a computer-aided method that comprises alternating between one or more of navigational tasks and measuring, qualification, and quantification tasks of a clinical task in accordance with defined simple click style user interactions. The defined simple click style user interactions are based upon a domain knowledge that includes details of an anatomy and details of a clinical measurement, quantification, or workflow of the clinical task associated with the anatomy. Responsive to execution of a simple click style user interaction within a current view, and further in accordance with a corresponding navigational task or measuring, qualification, or quantification task, the method transitions within the clinical task and its workflow between one or more of a first measurement point and a next measurement point within the current view or the current view and a next view.

However, the user still has to manoeuvre through a nested menu click-by-click to complete a desired workflow. Therefore, finding an answer to a posed question may still be cumbersome and important information may be overlooked by the user while clicking through the workflow.

### OBJECT OF THE INVENTION

Therefore, it is an object of the present invention to provide a method and apparatus capable of efficiently providing the user with the desired information in less time and with higher quality.

The invention solves the above problem with a computer implemented method for displaying visualizable data including the features of claim 1, a computer program including the features of claim 10 and with a user interface including the features of claim 11.

### SUMMARY OF THE INVENTION

According to an aspect of the present invention a computer implemented method for displaying visualizable data including at least one structure of interest is provided, the method comprising:
- receiving a user input via a user interface, the user interface allowing the user to select
   - at least one structure, and/or
   - at least one view of at least one structure, and/or
   - at least one function and/or
   - at least one time-interval and/or
   - at least one time-dependent measurement, and/or
   - a reading stack and/or
   - a modality
- with a single command issued by the user so as to generate user input data,
- determining a presentation sequence of the visualizable data based on the user input data, and
- displaying at least some of the visualizable data according to the presentation sequence of the visualizable data.

In contrast to the prior art, in the inventive method, the user may generate user input data by a single interaction with the user interface. Based on the user input data, the available visualizable data may be resorted such that the important data for answering the user s specific question may be directly (e.g., prominently) displayed to the user. The user may generate user input data corresponding to the question of the user without preforming any transfer performance between a natural thinking of the user and a technical approach of a program (e.g., the inventive method). Accordingly, the user may adjust the kind of displaying the visualizable data intuitively based on the specific question to be answered or task to be executed. In other words, a natural way of thinking of the user is realized during the user's interaction with the user interface. As a result, the assessment of the user may be accelerated. Further, the chances of overlooking some important visualizable data may be reduced or even completely prevented.

The visualizable data may initially be in an arbitrary order or sequence. For example, the order of the visualizable data may correspond to a recording or uploading sequence of the visualizable data. The visualizable data may be data that may be provided as a digital data, e.g., in DICOM standard. Further, the visualizable data may comprise image data. In addition, the visualizable data may comprise further information corresponding to the image data such as information of the structure(s) depicted within image data, measurement results, measurements, and/or previously made assessments. The visualizable data may comprise a three-dimensional array of voxels, each voxel containing a grey scale value. In particular, the visualizable data may be 3D medical image data. Such 3D medical image data have typically been obtained from a field of view containing the structure of interest using a medical imaging modality, such as MR, computed tomography (CT), positron emission tomography (PET) or ultrasound (US), for example. The structure of interest may be an anatomical structure. When the anatomical structure is the heart, ultrasound and in particular transoesophageal echocardiography (TEE) may be advantageously used to obtain the visualizable data. For example, the 3D medical image data may be acquired over a time span so as to obtain four-dimensional (4D) medical image data, wherein the fourth dimension is the time. In this case, one 3D image from the 4D medical image data may be also called a "frame" or "image" in the following. The 3D images may be acquired with a frame rate of, e.g., 5 - 100, preferably 20 - 60 images per second, so as to allow a smooth representation of a dynamically moving structure of interest. The time period is usually at least one cycle of the cyclical movement, e.g., at least one heartbeat. The 3D medical image data can be received directly from a modality that has acquired the medical image data or from a clinical image database. Further, the visualizable data may comprise two-dimensional (2D) image data. Moreover, the visualizable data may comprise a combination of 2D, 3D and/or 4D data.

The structure of interest may be an anatomical structure or a part of an industrial machine. The structure of interest may comprise a plurality of sub structures. For example, the structure of interest may be a human or animal heart, lung, liver, kidney or any other anatomical structure. For example, the visualizable data may comprise information of several anatomical structures of a human. The structure of interest may be one of these anatomical structures.

The step of receiving a user input may be triggered by a user s interaction with the user interface. The user interface may be a human-machine interface between the user and a system configured to execute the above method. The user interface may be a device which is configured to translate a physical interaction by the user therewith (e.g., a click with an input device, a gesture of the user or a finger tap, etc.) to a signal which may be the user input on the side of the system. In other words, the user interface may be configured to interpret a user action so as to generate user input data.

The user interface may be a device which is configured to dynamically display a menu and to receive a physical interaction by the user. The physical interaction may be the single command executed by the user. The dynamic display of the menu may be defined by adapting the menu to the available visualizable data. In other words, in case the visualizable data are medical data, the menu may be displayed in a different way as compared to a case in which the visualizable data are industrial data. For example, predefined analysis such as a reading stack (option f)) or function (option c)) may be differently defined. Further, the user interface may translate a physical interaction by the user therewith into the user input data (i.e., into a signal). For example, the user interface may be a touch-sensitive display. The user interface may be part of a system which is configured to execute the method. The system may be a computer.

Further, the user interface may be configured to offer the user at least one of the selections a) to g) such that the user may select one of them with a single interaction between the user and the user interface. The at least one of the selections a) to g) may be offered to the user by a menu. The menu may comprise at least one push button. Preferably, there are provided a plurality of push buttons. Each push button may be identified by a schematic graphic indicative of any one of the options a) to g). The options a) to g) may correspondent to a question and/or task to be answered. This way, the user may easily grasp which button has which function. That is, the user may choose at least one of the options a) to g) directly based on his or her question or task to be answered without translating the question or task in a technical way. Accordingly, the workflow may be realized intuitively. In the following the options a) to g) will be described in detail.

According to option a), the user interface may allow the user to select at least one structure. The structure may be the structure of interest included in the visualizable data. For example, the structure may be a human or animal heart, lung, liver, kidney or any other anatomical structure. Further, the structure may be a substructure of a larger structure included within the visualizable data. For example, the structure may be a ventricle of the human heart, a chamber of the human heart and so on. Accordingly, the structure that is the subject of the posed question or task may be selected directly and specifically by the user. Further, the push button indicative of the structure may be visualized by a schematic graphic identifying the respective structure. In addition, the structure may be an industrial part such as a gear, clutch, machine, etc.

According to option b), the user interface may allow the user to select at least one view of at least one structure. The view may be the viewing direction in which the visualizable data and/or structure is depicted. In particular, the view may be a sectional view of a 3D visualization. Further, the view may be a MPR plane. In addition, the view may be a top view, a side view, a front view or a bottom view of a structure of interest, for example. In the clinical field, the view may be an apical four-chamber view, an apical three-chamber or an apical two chamber view of the heart, for example. In addition, the view may be a five-chamber view, an inferior vena cava, long axis right ventricular inflow, parasternal short axis aortic view, parasternal short axis apical view, parasternal short axis mitral view, parasternal short axis papillary muscle view, right ventricular outflow tract view, special TEE views, subcostal four chamber-view, suprasternal view of the aortic arch, transthoracic standard parasternal long axis or two chamber-view. Further, any other standardized views like iso-views may be chosen under option b). In addition, interrelated views may be collected in group of views. Accordingly, option b) may allow the user to select a plurality of views with one single command. For example, short axis views may be collected to one group of views. Similarly, long axis views may be collected to another group of views. In this case, the user input data may be indicative of all views contained in a selected group of views. This allows the user to select a variety of views tailored to the question or task at hand with a single command.

According to option c), the user interface may allow the user to select at least one function. The function may be a function of the structure of interest. For example, the function may be an impermeability of a heart valve. Further, the function may be an intended measurement to be executed within the visualizable data. For example, the measurement may be a length determination, a volume determination and/or an area determination. Moreover, the function may comprise a diagnostic issue or a pathology related to an examination to be carried out. In addition, the function may be an electrocardiogram of the heartbeat phase (systole) and/or the relaxation phase (diastole). In addition, any other functional content of the visualizable data may be defined under option c). Functional content may mean a visualization of any measurement parameter over time or specific motion sequence of a structure included within the visualizable data.

According to option d), the user interface may allow the user to select at least one time-interval. The time interval may be selected in case 4D visualizable data are available. Such data may be the movement of a structure of interest, for example. In the medical field, the time-interval may be a cycle of a heartbeat, for example. In addition, the time interval may be a diastole or a systole of the cardiac cycle. That is, content of option d) may overlap with content of the option c). However, by providing both (i.e., option c) and option d)), the user may intuitively select the option corresponding to his or her question or task in mind. Sometimes, selection one of option c) or d) may be more intuitive for the user than the other one. In this case, the user may obtain the same result by either selecting option c) or d). Further, the options c) and d) may be differently displayed to the user (e.g., by different graphical icons). Therefore, the workflow may be executed intuitively without the need to translate the user s question or task in technical commands.

According to option e), the user interface may allow the user to select at least one time-dependent measurement contained in the visualizable data. The measurement may comprise Doppler and/or B-mode information. The time dependent measurement may be a measurement at different points in time. For example, a comparison of a structure at different point in times is possible. As a result, a course of a disease may be monitored, for example. By being able to select a measurement, the user may be in a position to directly select what result the user needs to answer his or her question or task.

According to option f), the user interface may allow the user to select a reading stack. The reading stack may be a predefined workflow for performing a standard examination, for example. Such examination may comprise one or more measurements or measurement results. For example, the reading stack may be a Doppler measurement executed based on the visualizable data. Further, the reading stack may be a pathological approach. In addition, the reading stack may be a combination of views of one or more structures. That is, a reading stack may be used to assess an overall status of an anatomical structure. If the anatomical structure is the heart, the reading stack may include an apical 4-chamber view, Doppler information, and views of the ventricle, for example. Depending on the type of visualizable data, any other combination is possible. For example, a reading stack may be a sorting order of images of the visualizable data for a specific pathological question.

According to option g), the user interface may allow the user to select a modality. The modality may be the modality which is used to acquire at least a part of the visualizable data. The modality may be magnetic resonance imaging (MRI), computed tomography (CT), positron emission tomography (PET) and/or ultrasound (US). Therefore, the user may directly select the modality that they know will provide the appropriate visualizable data to answer their question or task.

At least some of the options a) to g) may overlap with one another and may thus lead to the same result (i.e., the same user input data). However, it may advantageous to display more options to the user, such that the user may select intuitively based on the user s question or task to be answered.

The single command issued by the user may be the trigger to generate the user input data. That is, the single command may be characterized in that only one interaction between the user and the user interface is necessary to generate the user input data. In more detail, the user interface may be configured such that the user is in a position to address his task or question regarding the visualizable data (e.g., a structure, an object or anything else described by the visualizable data) with one single command. The command may be a click using a pointing device, a tap with one or more fingers of the user, a gesture made by the user or any other action by the user which may be recognized by the user interface. Accordingly, in case of a clinical application of the present invention, the user may have a question relating to the dimensions of the aortic valve of a heart. Then, the user may use the user interface to click on a button relating to the structure of the aortic valve of the heart. Subsequently, the user input data is generated. Further, based on the user input data, the visualizable data is shown to the user (further details follow below). This allows the user to get visualizable data to work with in just one interaction. In other words, the user may not translate his or her question or task in a technical command to be inputted to the system, but may be able to input the task or question itself (e.g., by selecting at least one of the options a) to g)) to the user interface so as to obtain the desired data. Consequently, the workflow may be more intuitive and thus more efficient. Further, extensive training on the system to understand the underlying technical approach may be unnecessary to work with the system executing the above method.

The user input data may be an internal signal configured to indicate the user's selection of at least one of the options a) to g). In other words, the user input data may be indicative of the user's question or task to be answered by the visualizable data. For example, the user may have a question relating to a specific anatomical structure. Accordingly, the single command may be executed by the user using the user interface so as to select option a). Then, the user input data may be indicative of the specific anatomical structure being the structure of interest for the user. In another case, the user may have a question relating to a specific pathology and may issue a corresponding user input data via the user interface. In this case, the user input data may be indicative of the visualizable data which is necessary for the treatment of the pathology in question. In any case, the user input data may be the functional link between the specific user task or question entered into the user interface by the single command and the visualizable data required to answer the user's question or task. In other words, the user input data may be indicative of which visualizable data corresponds to the selection made by the user via the user interface. The transfer from the selection made by the user to the user input data may be realized by a control unit. The selection may be an input to a control unit and the user output data may be an output of the control unit. That is, the control unit may be configured to map the selection made by the user to the user output data that is indicative of the visualizable data required to satisfy the requirements of the selection. Accordingly, the user does not need to decide what visualizable data is necessary for his or her specific question or task, but may directly input the question or task via the user interface (i.e., by selecting at least one of the options a) to g)). Then, the control unit may be configured to translate the input (i.e., selection) into a technical request to determine the visualizable data needed to answer the user's question or task. Accordingly, the efficiency of the workflow may be significantly increased.

User input data may only be generated in case the user interacts with the user interface so as to select one of the options a) to g) to answer the user's question or task. That is, moving a pointer over a display of the user interface may not be considered a user command and may thus not generate user input data. The same may be true for adjusting configurations of the user interface such as brightness of a display or other system configuration. However, as long as the user interacts with the user interface in a way that relates to the user s question or task, this interaction may be considered as a command issued by the user. For example, scrolling through a menu so as to select at least one submenu to end up at a desired button to generate user input data may be considered as being a plurality of commands issued by the user. On the other hand, the present invention provides the feature that the user may generate the user input data by only one single command (i.e., by the selection of at least one of the options a) to g)).

Based on the user input data the presentation sequence of the visualizable data may be determined. In other words, the sequence in which the visualizable data may be provided to the user may be adjusted based on the user input data. Sequence may be the order from a first image (e.g., 2D, 3D or 4D) to any one of the subsequent images comprised in the visualizable data. For example, if the user input data specifies the human heart as a structure of interest and the visualizable data includes a plurality of images representing the entire human body or other parts of the human body, the order of the images included in the visualizable data may be determined such that the images including the heart are provided first. Accordingly, the user having input the single command may subsequently examine the images of interest first. In other words, the determination of the presentation sequence may comprise a resorting of the images included in the visualizable data. That is, the user may not have to scroll through a large number of images that do not match his question or task, but is offered the important images directly. This significantly reduces or even completely suppresses the risk of overlooking important images that are necessary to answer the user's question or task.

Further, the visualizable data may be displayed to the user in the previously determined sequence. That is, the user s command may be the input to a system configured to execute the above method and the visualizable data in the determined sequence may be the output of the system to the user. In addition, the visualizable data may be tailored based on the user input data. That is, the size of the visualizable data may be reduced to include only the necessary visualizable data to answer the user's question or task. Accordingly, a handling of the visualizable data may be facilitated. In addition, the user may work with less data which enables the user to work on less powerful computers, for example. In order to determine the visualizable data necessary for answering to the user s question or task, the visualizable data may include information of properties and/or content of the visualizable data. For example, the visualizable data may include images (e.g., 2D, 3D and/or 4D). In this case, the content of the images may be an information of what is depicted by the respective image. Further, properties of the image may be included in the visualizable data. The properties may be a resolution of the image, direction of the exposure axis (in what angle a probe was held during acquiring the image using US) and/or type of image (standardized images such as four-chamber view, three-chamber view, etc.). In addition, measurements previously performed using a specific image or images may be included within the visualizable data. Furthermore, a pathology correspondent to specific images of the visualizable data may be included within the visualizable data.

The displaying the visualizable data to the user may be realised on the same device which was previously used to input the user's command. For example, such device may be a touch sensitive display or the like. As a result, a system configured to execute the above method may have a compact size.

According to a further embodiment, the visualizable data is medical data including at least one anatomical structure, wherein the user interface may allow the user to select at least one anatomical structure, and/or, at least one view of at least one anatomical structure, and/or, at least one function and/or, at least one pathology, and wherein the medical data is preferably obtained by a multimodal examination.

The medical data may be stored and received from a database. Accordingly, a large amount of visualizable data of one patient under examination may be considered. In particular, in such database visualizable data may be stored that was acquired at different points in time. Accordingly, the user input data may comprise a time component. In other words, the user input data may be indicative of a point in time at which the visualizable data should be displayed to the user. For example, in some cases it may be important to compare older images with recently acquired images. Because the user input data may also comprise a time component this issue may be also considered by the determination of the sequence of the visualizable data. However, the visualizable data may be acquired simultaneously with the execution of the above method. That is, the method may be executed in parallel to an examination of a patient. Accordingly, the method may be also useable in the emergency medicine.

The detailed selections of the present embodiment may be additionally offered by the user interface to at least one of the above selections a) to g). Accordingly, the user s question or task may be addressed in more detail. The multimodal examination may be an examination using more than one modality. For example, the visualizable data may comprise data acquired by CT and acquired by US. Accordingly, the advantage of each modality may be used answering to the user s question or task.

According to a further embodiment, the method may further comprise detecting the content of the visualizable data, wherein the determination of the presentation sequence may be further based on the content of the visualizable data in that the at least one structure and/or the at least one view of the at least one structure and/or the at least one function is derived from the content of the visualizable data.

The detection of the content of the visualizable data may be a determinisation of what is depicted and/or represented by the visualizable data. The detection of content of the visualizable data may be executed the first time (e.g., in case the visualizable data are analysed the first time). Accordingly, the visualizable data may be assigned to a user input (e.g., to any one of options a) to g)). Further, the detection may be executed even if there is included some information in the visualizable data what is depicted by the visualizable data (e.g., in case the visualizable data was previously processed or the information were added to the visualizable data in another way). In this case, the previously made detection may be checked and/or completed. This may prevent a case in which an available detection is wrong or incomplete. The detection may be an automatic image classification and/or content detection. The detection may be executed previously to receiving the user input. The step of detecting the content of the visualizable data may comprise labelling of images (2D, 3D or 4D) included in the visualizable data. The label may comprise information such as "apical 2-chamber view containing the left ventricle and mitral valve".

According to an embodiment, the step of detecting content of the visualizable data is performed using an artificial intelligence algorithm. The artificial intelligence algorithm may compare the visualizable data to known images and/or content and may match the visualizable data to the content once there is a similarity between the known content and the visualizable data. Accordingly, the algorithm may be configured as a comparator. As a result, an efficient way of determining the kind and/or content of the visualizable data may be provided. The kind of visualizable data may be a detection of views (e.g., standard view such as 2-chamber view, 3-chamber view, 4-chamber view etc.). In addition, an acquisition angle (e.g., camera angle) may be determined.

The artificial intelligence algorithm may be an artificial neural network configured to detect content of the visualizable data. For example, the artificial neural network may be trained so as to classify visualizable data. Preferably, the method further includes training the artificial neural network. Accordingly, training input image data and corresponding training output data may be provided so as to train the neural network. Once the neural network is trained, it may provide an output including a classification of images previously inputted to the neural network. Using a neural network to determine the kind and/or content of the visualizable data may provide an improved or improvable classification process of the visualizable data. It was found that a convolutional neural network is particularly beneficial for classifying the content of visualizable data.

According to a further embodiment, the step of determining the presentation sequence of the visualizable data is further based on measurement data obtained by previously performed measurements in the visualizable data.

The visualizable data may comprise measurement data. That is, previously performed measurements may be assigned to one or more images (2D, 3D or 4D) of the visualizable data. The measurement data may be also considered in determining the presentation sequence. That is, in case the user s question or task may be answered by a previously executed measurement, the corresponding visualizable data (e.g., the image and the corresponding measurement data) may be arranged to be prominently displayed to the user. In addition, a radiologist normally performs standard measurements upon acquiring visualizable data. These standard measurements may also be considered as measurement data and may be included in the visualizable data. That is, the measurement data may also be used to classify the visualizable data. In other words, the measurement data may be also used to automatically identify objects, structures, environments etc. depicted by the visualizable data. Accordingly, the step of detecting content of the visualizable data may be improved.

According to a further embodiment, the step of determining a presentation sequence includes assigning the visualizable data to different hierarchy levels based on the relevance of the visualizable data to the user input data, and wherein the step of determining the presentation sequence may be further based on the hierarchy levels of the visualizable data.

In other words, the step of determining the presentation sequence may generate two, three, four or more relevance levels and assign the visualizable data to the respective relevance level. The first relevance level may comprise the visualizable data which is the most relevant to answer to the user s question or task. Accordingly, the first relevance level may be directly and prominently displayed to the user. This allows the user to see at a glance the most important visualizable data. In other words, the user does not need to scroll or browse through a lot of images to find the relevant one, for example. The other visualizable data may be assigned to the following relevance levels. If the other visualizable data have the same relevance to the user's question or task, there may be only one additional relevance level (i.e., two relevance levels in total). However, if the other visualizable data comprise more relevant and less relevant visualizable data, the other visualizable data may be subdivided in further relevance levels. For example, in case the user wants to analyse a specific anatomical structure, all visualizable data of said structure may be assign the first relevance level. For example, visualizable data including adjacent structures may be assigned to the following second relevance level. In addition, more distant structures may be assigned to the third relevance level. In this way, the user may easily overlook the visualizable data by their relevance for his or her question or task. That is, sometimes it may be useful to briefly examine the anatomical structures adjacent to the actual structure being examined. By applying the above method, the user may easily consider the surrounding of the user s region of interest. This makes holistic examination efficiently possible.

According to an embodiment, the method further comprising performing an automatic measurement of at least one feature of the at least one structure contained in the visualizable data.

The automatic measurement may be an image-based measurement, such as length and/or volume measurements. Such measurements may be comprised in the measurement data and may be included in the visualizable data. That is, the measurement may be shown within the respective images in which it was performed. The user may only approve or adjust the measurement. This helps to speed up the workflow further and also could help inexperienced users. The automatic measurement may be done by a computer. Further, the automatic measurement may also save clicks and this way save time. Furthermore, the automatic measurements may be used for review of previously done measurements. In other words, the user may receive an indication that the automatic measurement deviates (e.g., by a certain or predefined percentage) from a previously performed measurement. Accordingly, the user may carefully check the specific measurement and/or the image (2D, 3D or 4D) in which the measurement was performed. If used for review or reporting or when measurements already exist, these measurements may be used to increase sorting accuracy (i.e., for determining the presentation sequence of the visualizable data). In addition, failures are prohibited especially in cases where more than one user works with the same visualizable data.

According to an embodiment, the method further comprising evaluating the visualizable data so as to determine a status of the structure.

The status of the structure may be a condition of the structure. If the structure is a mechanical component, the status of the structure may be the degree of wear of the component. In this case, the step of evaluating may comprise comparing visualizable data at different points in time. That is, the progression of wear may be monitored during the evaluation. Such comparison may be performed automatically. Based on a total change of the component or structure between the different points in time, a status of the component may be determining. Alternatively, or additionally, the speed of change may be considered by determining the status of the component.

In case the structure is an anatomical structure, the status may be defined by tags like "aortic dysfunction" or "healthy patient". That is, the step of evaluating the visualizable data may comprise an assessment of the visualizable data so as to diagnose the status of the patient and/or of a structure of interest. For example, the volume of blood may be automatically determined and based on the result, it may be assessed whether the value is in a normal or abnormal region. Accordingly, the status of the structure or the patient may be determined. The step of evaluating may be executed prior to receiving the user input. Further, the determining of the presentation sequence may be further based on the status of the structure. Accordingly, the user's question or task to be answered may be addressed in a targeted manner by providing the most relevant visualizable data.

For example, the status of the heart as a structure of interest may define the severity of diastolic dysfunction. The evaluation may comprise the processing a decision tree. The decision tree for diastolic dysfunction may have a decision path which may be mapped in a workflow, which already supports the determining of the sequence of the visualizable data. The first step in the workflow may be a determination of the ejection fraction (EF). Depending on whether this is normal or reduced, the decision path may follow the decision tree for normal EF or a decision tree with reduced EF. In the decision tree for normal EF three different statuses may be defined. First, a normal diastolic function. Second, intermediate. Third, diastolic dysfunction. The decision tree with reduced EF may output four different statuses of the heart. That is, normal diastolic dysfunction, cannot determine diastolic dysfunction, grade II diastolic dysfunction or grade III diastolic dysfunction.

According to a further embodiment, at least some of the visualizable data is displayed directly after receiving the single command issued by the user.

In other words, there may be no further interaction between the user and the user interface necessary after the user has selected one of the options a) to g) to display the relevant visualizable data to the user. Therefore, the visualizable data may be quickly displayed to the user with only one interaction (e.g., one click) executed by the user to generate the user input. In other words, the user may find and pick the desired structure or view at a glance and with one click. Accordingly, the handling the visualizable data may exhibit an improved intuitiveness and may follow the natural way of thinking of the user.

According to another aspect, the invention provides a computer program comprising program code instructions which, when executed by a processor, enables the processor to carry out the above method. The computer program may be in any code, in particular a code suited for computer graphic applications, in particular for image-analysing programming.

In a further aspect, the invention is directed to a computer-readable medium comprising an above-defined computer program. The computer-readable medium may be any digital data storage device, such as a USB-stick, hard disk, CD-ROM, SD card or SSD card. Naturally, the computer program need not be stored on such a computer-readable medium to be supplied the customers, but may be downloadable via the internet.

According to a further aspect, the invention is directed to a user interface for controlling a display of visualizable data including at least one structure, the user interface comprising:
- a display for displaying a map menu including at least one selection zone including at least one selectable item,
- an input device configured to allow a user to select a selection zone and/or a selectable item so as to generate the user input data with a single interaction between the user and the user interface, and
- a control unit configured to determine a presentation sequence of the visualizable data based on the user input data, and to display the visualizable data based on the presentation sequence of the visualizable data.

The user interface may include a display configured to display the map menu to the user. The display may be also configured to display the visualizable data. However, the visualizable data may be also displayed on another display. For example, the display may be a touch-sensitive display. Accordingly, the display may be used to receive the user s command and to show the visualizable data to the user. Accordingly, the user interface may be compact. The user interface may be part of a computer, tablet, workstation or any other data processing unit. Therefore, the user interface may be used in various technical fields.

The map menu may be a presentation of several selectable options (e.g., a) to g)). All available selectable options may be presented to the user at once. This means that the user may see all available selection options at a glance. The user therefore does not need to scroll or search for a desired option in hierarchically deeply nested menus. In other words, the display may be configured to show the user all available selectable options at once (i.e., all available selectable options may be presented in parallel). The map menu may be designed in a way that allows to identify the wished structure (e.g., left ventricle) or view (e.g., parasternal long axis view) or function (e.g., diastolic function) fast and all within one plain flat menu. The content of this kind of menu may be assimilated without scrolling or page turning. And, additionally, the selection may be made with one single click. This may allow optimal workflow and at the same time precise measurements. In more detail, the intended anatomical structure (or view or function or any other option a) to g)) may be selected first, and then the visualizable data may be presented in an adapted (i.e., resorted) presentation sequence to the user afterwards. This selection of the anatomical structure or view may be done with one click within the map menu enabling the user to precisely pick what he or she intends to measure or evaluate. The map menu may be a flat map of an anatomical "landscape". That is, flat map may mean that all available options may be presented at once to the user. The anatomical landscape may be implemented in case the user interface is used in the medical field. In this case, the anatomical landscape may describe a variety of different definitions in the anatomical context. For example, the anatomical structure, the measuring method, the pathology, the diagnostic question, intended measurements, etc. Since the map menu may be configured so as to be completely presented to the user at once, the map menu may be referred to as a mini-map menu. According to one aspect, the main idea is to provide a mini-map that allows the user to select different types of information simultaneously with a single click.

The at least one selection zone may include at least one selectable item. That is, the selectable item may be a sub selection of the selectable item. In other words, the selection zone may be a superordinated selection with respect to the selectable item. Accordingly, the user may choose either the broader selection (i.e., the selection zone) or may choose the limited selection by directly selecting the selectable item. The selection zone may be indicative of user input data that considers a larger amount of visualizable data to be important to the user than the selectable item. Therefore, the selectable item may provide a more accurate selection of the visualizable data than the selection zone. Even though the map menu presents the user with all the choices at once, the user may still select a precisely defined option to answer his or her question or task. Here, a map menu having two levels of defining an option to be selected is described (i.e., the selection region and the selectable item). However, more levels may be provided to define an option. That is, the selectable item may also contain at least one lower-level selectable element, and so on. The selection zones may be identified by a borderline provided around the selection zone. Within the borderline the selectable items may be graphically depicted. For example, a heart may be schematically depicted by line drawing (i.e., a structure). Further, a Doppler measurement may be depicted by a colour flow (i.e., a function). Moreover, a diastole and/or systole may be depicted by a schematical electrocardiogram in which the respective part (i.e., diastole or systole) is emphasized. In this way, any selectable item and/or selection zone may be schematically depicted in the map menu. Due to the graphical visualization the user may intuitively navigate to the desired option. Further, the user interface may be used internationally without the need of translating any written identification of the selectable items. Therefore, the implementation of the user interface may be simplified.

The input device may be a pointing device such as a computer mouse, trackball, trackpad, etc. However, the input device may be also realized by the display, in case the display is a touch sensitive display and the user may issue a user command by taping onto the display (e.g., with the user s finger). In addition, the input device may be a camera which may be configured to recognise movements and/or gestures made by the user. Accordingly, the user interface may be implemented in a variety of existing systems, from a desktop computer to a tablet to virtual environments or augmented reality environments.

The control unit may be a processing device configured to receive input data, process the data and to output command and/or output data. The control unit can be a remote control unit. That is, the control unit does not have to be located in the same place as the display. Accordingly, the method may be performed remotely. The control unit may be centrally located in a server, and the display and the input device may be located at the location where the user operates the input device. As a result, more than one user may work with the user interface at the same time.

According to an embodiment, the input device is configured to allow a pointer to be navigated by the user to hover over the map menu and to generate the user input data based on a current position of the pointer within the map menu, when a single command is issued by the user.

The single command may be a click, a tap, a gesture or any other recognizable action performed by the user. For example, the user may navigate a pointer within the map menu to a desired selection zone or selectable item, and may there activate the pointing device (e.g., clicking a computer mouse) to issue the command. As described above, the single command is sufficient to generate the user input data.

According to a further embodiment, the map menu includes a plurality of selection zones,
wherein each selection zone includes at least one selectable item,
wherein the at least one selectable item of a selection zone relates to
   - at least one structure, and/or
   - at least one view of at least one structure, and/or
   - at least one function and/or
   - at least one time-interval and/or
   - at least one time-dependent measurement, and/or
   - a reading stack and/or
   - a modality.

In other words, there may be provided a plurality of selection zones each including at least one selectable item. The selectable options a) to g) may be characterized as outlined above. A selection zone may be representing a generic type of any option according a) to g). For example, one selection zone may define views (i.e., a group of views). The selectable items within this selection zone may define specific views. Further, a selection zone may define short axis views. In the selectable items in said selection zone, the short axis views may be further assigned to specific structures or sub structures, for example. Accordingly, the orientation for the user in the map menu is facilitated and thus the workflow may be further accelerated.

According to an embodiment, the map menu includes a plurality of selectable items, and wherein the control unit is configured to at least partially highlight a selectable item or selectable items and/or selection zones when the pointer hovers over a corresponding other selectable item and/or selection zone.

For example, the user may move a pointer over the map menu (i.e., over the selection zones and/or the selectable items). As described above, the selection zones and/or the selectable items may be schematically depicted by line drawings. In case the user operates the pointer via the input device such that the pointer hovers over a sub part of the structure of interest, all other selectable items and/or selection zones relating to said sub part may be highlighted. A selection zone may be highlighted by making the border line bold, by shading and/or by depicting it in a different colour. A selectable item may be highlighted by depicting it with a different line thickness, shading or any other suitable means. For example, if the user hovers the pointer over the left ventricle in a selectable item associated with a selection zone that defines structure, the selectable item representing the left ventricle associated with a different selection zone that defines short-axis views may be highlighted. In this way, the user may intuitively recognize his or her options to select the correct option to answer the user s question or task.

According to a further embodiment, the map menu is at least partly configured as a flat map of structural landscape, preferably without any text.

As defined above, the map menu may be depicted to the user such that the user may see all available options at once. Therefore, no searching or multiple clicking through menus is necessary. The waiver for text may provide the advantage that the user may recognize his or her options intuitively. In addition, the menu can be presented more clearly, since no textual descriptions are required to define the options. In addition, the user interface may be easily used across language barriers, as each user can recognize their options without having to master a specific language.

According to one embodiment of the present invention, the invention may be implemented in echocardiographic diagnosis. First a map menu is depicted to the user (step one). This map enables the user to select either a view plane, or a group of views or a function or reading stack or an anatomical structure with one click. This map menu fits within one page, so no scrolling or turning pages is needed. When hovering over the map menu, the selected structure, view etc. may be highlighted. This way, by clicking inside or outside of an icon, close or far to it, on the wall or inside the volume, the user can select different kinds of information. However, the map menu may have a different look and/or way of function. The point is, that by one click the user may select between anatomical structures as well as view planes, which makes a difficult, deeply nested menu unnecessary. This helps to save time and facilitates the workflow.

Then in a second step the visualizable data may be resorted based on the previously made choice by the user. To accomplish this, some sort of automatic image classification may be implemented, e.g., detecting the visible structures in the visualizable data using a convolutional neural network. This way, every image of the visualizable data obtains a label (e.g., "apical 2-chamber view containing left ventricle and mitral valve"). This label may then be used to decide, whether an image fits a user-selection or not. For example, the user may select the anatomical structure "left ventricle". Then all images containing this "left ventricle" may be sorted to the beginning in the graphical user interface. This may be images labelled with "apical 2-chamber" or "apical 4-chamber" or "apical 3-chamber" or other kinds of views

(i.e., view planes) that contain this anatomical structure. Assuring to have the same order of view planes (e.g., 2-chamber followed by 3-chamber followed by 4-chamber) shown in this re-ordered view for every new study (i.e., collection of clips of a patient) further improves usability. All other images (which do not fit the selection) will still be visible to the user, but come after the selected ones in unchanged order. For example, the user may also select "apical 2-chamber" as desired object of interest. In this case, all acquired apical 2-chamber views may be put to the beginning of the sorted study. To prevent errors and/or to make the handling more intuitive, those 2-chamber views may be followed by 3-chamber and 4-chamber views, which are very close optically to the desired 2-chamber view. For example, the user may select a group of views, e.g., "apicals" which may lead to all apical views being shown at the beginning, followed by all other images of the study. For example, the user may select a function, e.g., "diastolic function", which may lead to selection of all images that are needed to evaluate diastolic function. Those are then placed at the beginning.

Basically, any reading stack (meaning a combination of acquired images/structures) may be defined and integrated into such a map menu. The resorting helps save time and also leads to a more structured workflow, decreasing the risk of overlooking images which could be important for a diagnosis. If a suboptimal image is taken for measurements, the resulting values might be wrong and the diagnosis based on those measurements as a consequence might be wrong, too, leading to wrong treatment.

In a third step, automatic measurements may be implemented. These may be in the form of image-based measurements, such as length and/or volume measurements, as well as general tags like "aortic dysfunction" or "healthy patient". Such measurements may be shown within the images and the user only needs to approve or adjust them. This helps to speed up the workflow further and also could help inexperienced users. A different implementation option would be to use this invention for review or reporting - or in whatever use case, where measurements already exist from the beginning. Then those measurements may be used to improve the order of step two. Moreover, the measurements may be used to pre-select reading stacks/views/structures for review. For example, starting from within a medical report, the user may select the fitting "parts" of the map menu, which may lead to a certain entry in the report. So, an "ejection fraction" measurement may include a link to the "apicals" selection, to show again the images, which were crucial for this value.

Individual features of the embodiments described above may be combined with other embodiments or with other features of other embodiments to form new embodiments. The effect mentioned in connection with each individual feature also applies to such new embodiments. Furthermore, the advantages and configurations mentioned in connection with the method also apply to the device and vice versa.

### BRIEF DESCRIPTION OF THE DRAWINGS

Useful embodiments of the invention shall now be described with reference to the attached figures. Similar elements or features are designated with the same reference signs in the figures. In the figures:
Fig.. 1 shows schematically an interaction between the user and the method according to an embodiment of the present invention;
Fig.. 2 shows a schematical flow diagram of a method according to an embodiment of the present invention;
Fig. 3shows a map menu according to an embodiment of the present invention;
Fig. 4 shows a map menu according to another embodiment of the present invention;
Fig. 5 shows a map menu according to an embodiment of the present invention, and
Fig. 6 shows a user interface according to an embodiment of the present invention.

### DETAILED DESCRIPTION OF EMBODIMENTS

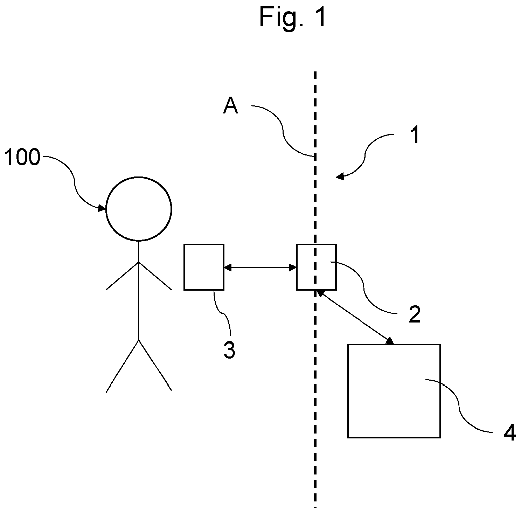

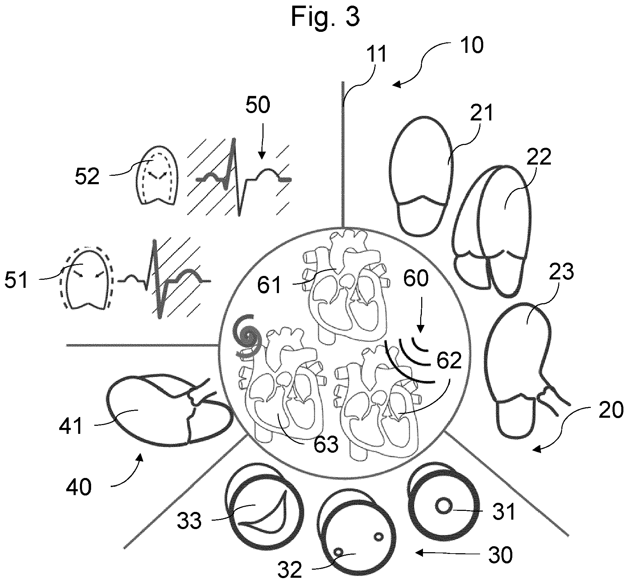

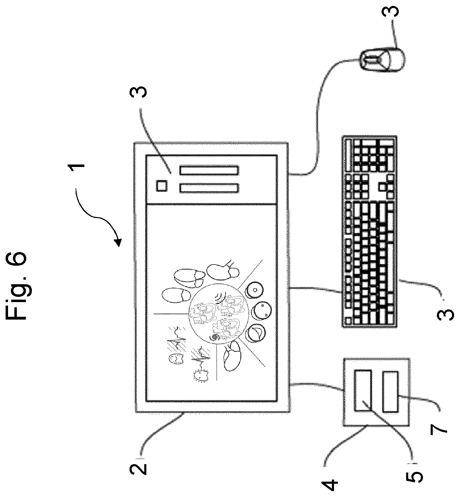

Fig. 1 shows a schematically an interaction between a user 100 and a user interface 1 according to an embodiment of the present invention. The user interface 1 comprises a display 2, an input device 3 and a control unit 4. The display 2 is configured to display a map menu 10 (refer to Fig. 3). In one embodiment, the display 2 is a touch sensitive display. In addition, the display 2 is configured to display visualizable data. In one embodiment, the visualizable data are medical image data. The medical image data comprises 2D image data, 3D image data and 4D image data. The input device 3 is configured to be operated by the user 100 so as to communicate with the user interface 1. In the present embodiment, the input device 3 comprises a computer mouse, a keyboard and virtual buttons on the display 3 (refer to Fig. 6). The control unit 4 is a processor configured to process user input data and to determine a presentation sequence of visualizable data (to be explained below).

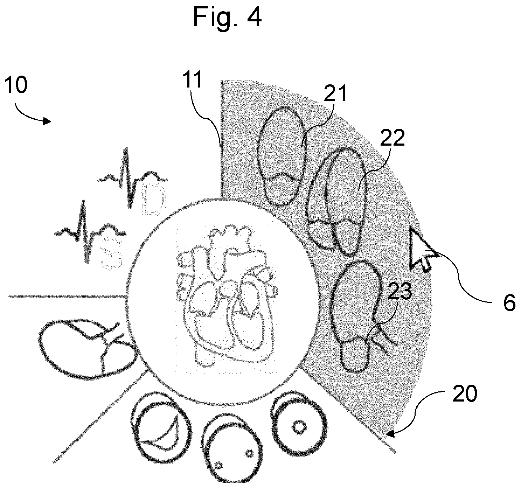

According to an embodiment of the present invention, visualizable data are provided. In one embodiment the user interface 1 comprises a storage 5 (refer to Fig. 6) in which the visualizable data are stored. In another embodiment, the user interface 1 is connected with an external data base in which the visualizable data is stored. In any case, the user 100 operates the input device 3 so as to move a pointer 6 (refer to Fig. 4) over the map menu 10. Once the user 100 reaches the desired option, the user 100 activates the pointing device 3 so as to choose an option of the map menu 10 at which the pointer 6 is currently positioned (details of the map menu will be described below). In more detail, the user may choose and select one or more of
- at least one structure, and/or
- at least one view of at least one structure, and/or
- at least one function and/or
- at least one time-interval and/or
- at least one time-dependent measurement, and/or
- a reading stack and/or
- a modality.

Accordingly, the user input data is generated. In other words, the user generates the user input data by one single command. Fig. 1 shows a boundary line A, which shows the boundary between a user side and a user interface processing side. That is, the left side in Fig. 1 is the user side and the right side in Fig. 1 is the user interface processing side. To generate the user input data, only one single command by the user is necessary to cross the boundary line A. In other words, only one interaction between the user 100 and the control unit 4 of the user interface 1 is necessary to generate the user input data. In other words, the user only once issues a single command to the control unit 4 of the user interface 1. The user input data is indicative of the selection made by the user in the map menu 10.

Based on the user input data, the control unit 4 determines a presentation sequence of the visualizable data. Then, the visualizable data is shown to the user via the display 2 in the determined presentation sequence.

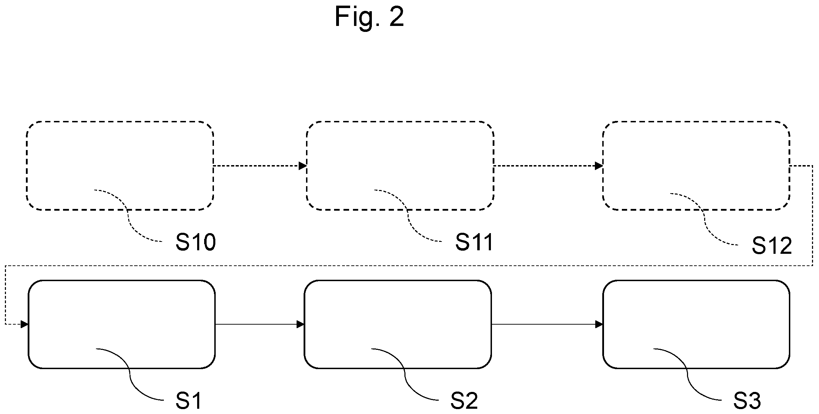

Fig. 2 is a schematical flow diagram showing the flow of the method according to an embodiment of the present invention. In one embodiment, in a first step (step S1) a user input is received via the user interface 1, the user interface allowing the user to select at least one of options a) to g). This step is characterized in that the user input is received by a single command issued by the user. That is, only one interaction between the user and the user interface 1 (especially between the user and the control unit 4) is necessary to receive the user input. The user input is then used to generated user input data. Then, in a second step (step S2) the presentation sequence of the visualizable data based on the user input data is determined. In particular, the control unit 4 receives the visualizable data and the user input data and determines the presentation sequence. In other words, the control unit 4 resorts the visualizable data so as to correspond to the user input data. That is, the visualizable data important to the user will be arranged so as to be prominently (i.e., at first) displayed to the user in the next step. Subsequently, in the next step (step S3) the at least some of the visualizable data is displayed to the user 100. That is, the visualizable data is displayed to the user 100 via the display 2 in the determined presentation sequence. Accordingly, the user 100 is first directly shown the part of the visualizable data that is particularly important to him.

In another embodiment, prior to step S1 a step S10 is performed. In step S 10 the content of the visualizable data is detected. Further, the determination of the presentation sequence of step S2 is further based on the content of the visualizable data in that the at least one structure and/or the at least one view of the at least one structure and/or the at least one function is derived from the content of the visualizable data.

In a still further embodiment, the method comprises a step S 11 in which an automatic measurement of at least one feature of the at least one structure contained in the visualizable data is performed. The measurement is an image-based measurement. The result of the measurement is contained in the visualizable data. Accordingly, the result is also shown to the user in step S3.

In a still further embodiment, the method comprises a step S12 in which the visualizable data is evaluated so as to determine a status of the structure. The status of the structure is then also displayed to the user 100 in step S3.

It is to be noted that the steps S10, S11 and S12 are not necessarily needed to perform the method according an embodiment of the invention. For example, the visualizable data may be tagged (e.g., during the acquiring process) such that no detection is necessary to identify the images contained in the visualizable data. Further, all steps S10, S11 and S12 may be provided or only one or two thereof.

Fig. 3 shows a map menu 10 according to an embodiment of the present invention. In the map menu 10 depicted in Fig. 3, five selection zones are provided. The selection zones are at least partly surrounded by a boundary line 11. A first selection zone 20 is assigned to a group of views. A second selection zone 30 is assigned to short axis views and structures. A third selection zone 40 is assigned to a long axis view and a structure. A fourth selection zone 50 is assigned to a time interval and time-dependent measurements. A fifth selection zone 60 is assigned to structures, modalities and functions.

The first selection zone 20 includes three selectable items. The first selection zone first selectable item 21 is an apical-two-chamber view and structure. The first selection zone second selectable item 22 is an apical-four-chamber view and structure. The first selection zone third selectable item 23 is an apical-three-chamber view and structure.

The second selection zone 30 includes three selectable items 31, 32, 33. The third selection zone 40 includes one selectable item 41. The fourth selection zone 50 includes two selectable items 51, 52. The fourth selection zone first selectable item 51 represents the systole. The fourth selection zone second selectable item 52 represents the diastole. The fifth selection zone 60 includes three selectable items 61, 62, 63. The fifth selection zone first selectable item 61 represents the structure of interest. The fifth selection zone second selectable item 62 represents a Doppler anatomy. The fifth selection zone third selectable item 63 represents colour flow anatomy.

The user 100 may select one of the selectable items or the whole selection zone. Accordingly, the user input data may be generated so as to be indicative of all selectable items included in the selected selection zone or of the specific selectable item selected.

In Fig. 4 the map menu 10 according to another embodiment is depicted. The present embodiment corresponds essentially to previously described embodiment with the exception that some selectable items and selection zones are visually realized in a different way. For simplicity, not all reference signs are shown repeatedly in Fig. 4. Further, in Fig. 4 the pointer 6 is depicted which is hovering over the map menu 10. Currently, the pointer 6 is positioned within the first selection zone 20. In the present embodiment, the first selection zone 20 is highlighted. Accordingly, the user 100 may easily recognize that now the first selection zone 20 is ready to be chosen by a click, for example. If the user operates the pointing device 6 so as to click, the user input data is generated based on the user interaction. In this case the user input data is indicative of the first selection zone 20 which is a group of views. Accordingly, the visualizable data will be resorted so as to put the group of views assigned to the first selection zone 20 in front of the visualizable data (i.e., to be prominently presented to the user).

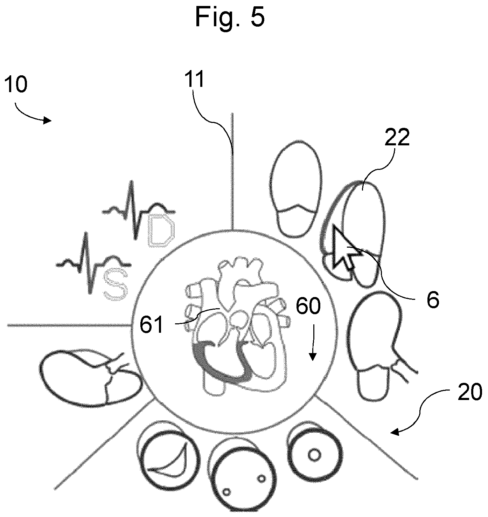

In Fig. 5 the map menu 10 of Fig. 4 is depicted, wherein the pointer 6 is positioned at a different position. In more detail, the pointer 6 is positioned over the right ventricle of the first selection zone second selectable item 22. In this case, all right ventricles of other selection zones and selectable items are highlighted. In particular, in the fifth selection first selectable item 61 (which represents the structure), the right ventricle is highlighted. That is, by hovering over the map menu 10, all corresponding features or sub features depicted by the selection zones and/or by the selectable items may be highlighted. In other words, the map menu 10 is a dynamical map menu adjusting its appearance depending on the position of the pointer 6 within the map menu 10. According to a further embodiment, the selection zones and/or the selectable items that are highlighted (it may be also possible to highlight only a portion of a selection zone and/or the selectable item) may be selected by the user input. That is, even beyond the borders of one selection zone or one selectable item, a structure or feature may be selected by the user. For example, by hovering over a selectable item indicative of right ventricular information all selectable items referring to right ventricular information may be highlighted. Further, by issuing the user input (i.e., by clicking on the highlighted selectable item), all visualizable data containing right ventricular information may be considered as being important in determining the presentation sequence (i.e., being shown first to the user).

Fig. 6 shows a user interface 1 according to an embodiment of the present invention. In the present case the above method is executed by a computer. The display 2 is a touch-sensitive monitor. Accordingly, virtual buttons as an input device 3 are provided. In addition, the input device comprises a computer mouse and a keyboard. The control unit 4 comprises a storage 5 and an interface to an external database 7. As a result, visualizable data may be received by the user interface 1 and may be locally saved.

### REFERENCE SIGNS

- 1: user interface
- 2: display
- 3: input device
- 4: control unit
- 5: storage
- 6: pointer
- 7: interface
- 10: map menu
- 11: boundary of selection zones
- 20: first selection zone
- 21: first selection zone first selectable item
- 22: first selection zone second selectable item
- 23: first selection zone third selectable item
- 30: second selection zone
- 31: second selection zone first selectable item
- 32: second selection zone second selectable item
- 33: second selection zone third selectable item
- 40: third selection zone
- 41: third selection zone first selectable item
- 50: fourth selection zone
- 51: fourth selection zone first selectable item
- 52: fourth selection zone second selectable item
- 60: fifth selection zone
- 61: fifth selection zone first selectable item
- 62: fifth selection zone second selectable item
- 63: fifth selection zone third selectable item
- S1: first step
- S2: second step
- S3: third step
- S10: fourth step
- S11: fifth step
- S12: sixth step
- 100: user
- A: boundary line

## Claims

1. Computer implemented method for displaying visualizable data including at least one structure of interest, the method comprising:
- receiving a user input via a user interface (1), the user interface (1) allowing the user (100) to select
- at least one structure, and/or
- at least one view of at least one structure, and/or
- at least one function and/or
- at least one time-interval and/or
- at least one time-dependent measurement, and/or
- a reading stack and/or
- a modality
- with a single command issued by the user (100) so as to generate user input data,
- determining a presentation sequence of the visualizable data based on the user input data, and
- displaying at least some of the visualizable data according to the presentation sequence of the visualizable data.

2. Method according to claim 1,
wherein the visualizable data is medical data including at least one anatomical structure,
wherein the user interface (1) allows the user (100) to select at least one anatomical structure, and/or, at least one view of at least one anatomical structure, and/or, at least one function and/or, at least one pathology, and
wherein the medical data is preferably obtained by a multimodal examination.

3. Method according to claim 1 or 2 further comprising:
- detecting the content of the visualizable data, wherein the determination of the presentation sequence is further based on the content of the visualizable data in that the at least one structure and/or the at least one view of the at least one structure and/or the at least one function is derived from the content of the visualizable data.

4. Method according to claim 3, wherein the step of detecting content of the visualizable data is performed using an artificial intelligence algorithm.

5. Method according to any of the preceding claims, wherein the step of determining the presentation sequence of the visualizable data is further based on measurement data obtained by previously performed measurements in the visualizable data.

6. Method according to any of the preceding claims,
wherein the step of determining a presentation sequence includes assigning the visualizable data to different hierarchy levels based on the relevance of the visualizable data to the user input data, and
wherein the step of determining the presentation sequence is further based on the hierarchy levels of the visualizable data.

7. Method according to any one of the preceding claims, further comprising:
- performing an automatic measurement of at least one feature of the at least one structure contained in the visualizable data.

8. Method according to any one of the preceding claims, further comprising:
- evaluating the visualizable data so as to determine a status of the structure.

9. Method according to any one of the preceding claims, wherein at least some of the visualizable data is displayed directly after receiving the single command issued by the user.

10. Computer program comprising program code instructions which, when executed by a processor, enables the processor to carry out the method of any one of the preceding claims.

11. User interface (1) for controlling a display of visualizable data including at least one structure, the user interface comprising:
- a display (2) for displaying a map menu (10) including at least one selection zone (20,30,40,50,60) including at least one selectable item (21,22,23,31,32,33,41,51,52,61,62,63),
- an input device (3) configured to allow a user (100) to select a selection zone (20,30,40,50,60) and/or a selectable item (21,22,23,31,32,33,41,51,52,61,62,63) so as to generate the user input data with a single interaction between the user (100) and the user interface (1), and
- a control unit (4) configured to determine a presentation sequence of the visualizable data based on the user input data, and to display the visualizable data based on the presentation sequence of the visualizable data.

12. User interface (1) according to claim 10, wherein the input device (3) is configured to allow a pointer (6) to be navigated by the user (100) to hover over the map menu (10) and to generate the user input data based on a current position of the pointer (6) within the map menu (10), when a single command is issued by the user (100).

13. User interface (1) according to claim 10 or 11,
wherein the map menu (10) includes a plurality of selection zones (20,30,40,50,60),
wherein each selection zone (20,30,40,50,60) includes at least one selectable item (21,22,23,31,32,33,41,51,52,61,62,63),
wherein the at least one selectable item (21,22,23,31,32,33,41,51,52,61,62,63) of a selection zone (20,30,40,50,60) relates to
- at least one structure, and/or
- at least one view of at least one structure, and/or
- at least one function and/or
- at least one time-interval and/or
- at least one time-dependent measurement, and/or
- a reading stack and/or
- a modality.

14. User interface (1) according to claim 10 or 12, wherein the map menu (10) includes a plurality of selectable items (21,22,23,31,32,33,41,51,52,61,62,63), and wherein the control unit (4) is configured to at least partially highlight a selectable item or selectable items (21,22,23,31,32,33,41,51,52,61,62,63) and/or selection zones (20,30,40,50,60) when the pointer (6) hovers over a corresponding other selectable item (21,22,23,31,32,33,41,51,52,61,62,63) and/or selection zone (20,30,40,50,60).

15. User interface (1) according to any one of claims 10 to 14, wherein the map menu (10) is at least partly configured as a flat map of structural landscape, preferably without any text.
